(19) Europäisches Patentamt
European Patent Office
Office européen des brevets

(11) EP 1 391 203 A1

(12) **EUROPEAN PATENT APPLICATION**
published in accordance with Art. 158(3) EPC

(43) Date of publication:
**25.02.2004 Bulletin 2004/09**

(21) Application number: **02769548.5**

(22) Date of filing: **08.05.2002**

(51) Int Cl.[7]: **A61K 31/5513**, A61P 35/00
// C07D401:04, C07M7:00

(86) International application number:
**PCT/JP2002/004482**

(87) International publication number:
**WO 2002/092096 (21.11.2002 Gazette 2002/47)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.05.2001 JP 2001141020**

(71) Applicant: **YAMANOUCHI PHARMACEUTICAL CO., LTD.**
**Tokyo 103 (JP)**

(72) Inventor: **YAMANO, M.,**
**c/o Yamanouchi Pharmaceutical Co.,**
**Tsukuba-shi, Ibaraki 305-8585 (JP)**

(74) Representative: **Bates, Philip Ian et al**
**Reddie & Grose**
**16 Theobalds Road**
**London WC1X 8PL (GB)**

# (54) ANTITUMOR AGENTS

(57) Since it has been confirmed that the antitumor agents of the invention containing as the active agent (3R)-N-(1-(1-tert-butylcarbonylmethyl)-2,3-dihydro-2-oxo-5-(2-pyridyl)-1H-1,4-benzodiazepin-3-yl)-N'-(3-(methylamino)phenyl)urea favorably inhibit the proliferation of cancer in various cancer-bearing mice and have a low toxicity, they are particularly useful in treating pancreatic cancer, intestinal cancer and gastric cancer. In case of using the medicaments of the invention particularly in polypharmaceutical chemotherapy, administration dose of an antitumor agent having strong side effects can be lowered, so that realization of a polypharmaceutical chemotherapy achieving a favorable antitumor effect and reduced side effects can be expected.

EP 1 391 203 A1

## Description

Technical Field

This invention relates to medicaments, particularly antitumor agents useful for treating pancreatic cancer.

Background of the Invention

In recent years, the rate of occurrence of cancers has been increasing steadily, and chemotherapy by various antitumor agents has been carried out for their treatment, together with surgical operation and radiotherapy. For example, polypharmaceutical chemotherapy which jointly uses antitumor agents such as 5-FU (5-fluorouracil) and derivatives thereof (tegafur, UFT, carmofur, etc.), MMC (mitomycin C), ADM (doxorubicin) and derivatives thereof (epirubicin, aclarubicin, etc.) and the like has been broadly carried out ("Cancer Chemotherapy Handbook, Method for using antitumor agents and anti-side effects policy" (edited by Issei Ogawa and Noboru Horikoshi; Igaku Shoin MYW; published October 20, 1994), pp. 235 - 323). However, from the viewpoint of their effects and strong side effects ( gastrointestinal toxicity, myelosugpression , renal toxicity, cardiac toxicity, alopecia, nausea, vomiting and the like), great concern has been directed toward the development of antitumor agents having more superior carcinostatic effects and less side effects.

Particularly, there are no chemotherapeutic agents capable of showing high efficacy on pancreatic cancer which is one of the intractable cancers, and a standard regimen is not established yet. Among various antitumor agents whose therapeutic results have recently been reported, a antimetabolite gemcitabine (GEM) showed a relatively good result, but it has side effects such as myelosuppression, fever, nausea, vomiting and the like. For the purpose of improving prognosis of pancreatic cancer, great concern has been directed toward the creation of a new type and effective antitumor agent (*Kagaku Ryoho-no Ryoiki* (Region of Chemotherapy), Vol. 14, S-1, pp. 275 - 280 (1998); and "Methods for Selecting and Using Antitumor Agents (Second Edition)" (edited by Issei Ogawa, published by Nankodo, October 15, 1999), pp. 41 - 42).

On the other hand, it has been reported that (3R)-N-(1-(1-tert-butylcarbonylmethyl)-2,3-dihydro-2-oxo-5-(2-pyridyl)-1H-1,4-benzodiazepin-3-yl)-N'-(3-(methylamino)phenyl)urea represented by the following formula which is the active ingredient of the medicament of the invention, (to be referred to as compound A hereinafter), has excellent selective gastrin/CCK-B receptor antagonism and, particularly, efficiently inhibits gastric acid secretion caused by gastrin *(J. Med. Chem.,* 1997, 40 (3), 331 - 341).

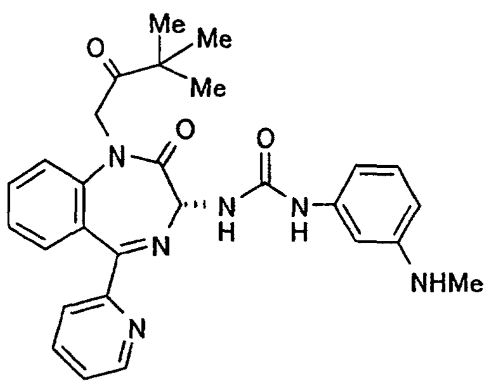

**Compound A**

Also, WO 93/16999 discloses medical compositions which act as a gastrin or CCK-B receptor antagonist and contain benzodiazepine derivatives of the following general formula (I) or salts thereof and these compounds as the active ingredients, particularly the aforementioned compositions which are medicaments for treating diseases induced by incompleteness of physiological functions controlled by gastrin. A racemic body of the compound A is disclosed in Example 57, but there is no disclosure on the compound A itself as an optically active substance.

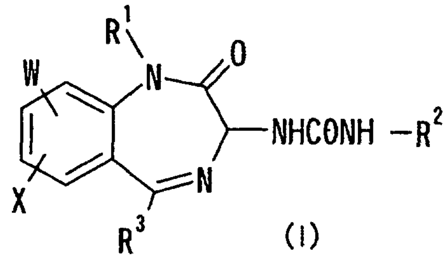

(I)

(In this formula, $R^1$ is $-CH_2CHOH(CH_2)_aR^4$, a ketone group - $CH_2CO$ $(CH_2)_aR^5$ or $-CH_2COC(CH_3)_2R^5$, a is 0 or 1, $R^4$ and $R^5$ are selected from alkyl and cycloalkyl groups and saturated heterocyclic groups whose hetero atoms are optionally substituted, (omission), $R^2$ and $R^3$ are independently selected from optionally substituted aromatic carbon rings and optionally substituted aromatic heterocyclic ring residues, and W and X are independently selected from halogen and hydrogen atoms and alkyl and alkoxy groups.)

In addition, the compound A is disclosed in Example 52 of WO 95/06040 which also discloses a synthetic method thereof and a medical composition containing said compound as the active ingredient, for use in the treatment of diseases induced by incompleteness of physiological functions controlled by gastrin.

In the aforementioned two patent documents, stomach and colon cancers are cited, in addition to gastric and duodenal ulcers, gastritis, reflux esophagitis and the like, as the diseases induced by incompleteness of physiological functions controlled by gastrin. However, these official gazettes disclose only binding affinity for CCK-B receptors and inhibitory action upon pentagastrin-stimulus gastric acid secretion in rats, and there is no illustrative disclosure on the action upon stomach and colon cancers.

There are reports stating that gastrin/CCK-B receptor is expressed in lung cancer and large bowel cancer cells, and gastrin increases proliferation of these cancer cells (*Am. J. Surg.*, **149**, 35 - 8, 1985; and *Arch. Sur.*, **124**, 470 - 2, 1989). Also, there is a report stating that a 1-methylphenacylbenzodiazepine derivative inhibited proliferation of human gastrin/CCK-B receptor-expressing murine cells by its binding inhibitory effect to the receptor (JP-A-8-40908).

However, to date there are no clinical reports showing usefulness of a selective gastrin/CCK-B receptor antagonist as an antitumor agent. Also, there are no reports which clearly support antitumor effect of a selective gastrin/ CCK-B receptor antagonist by tests using an animal model.

For example, when a glutamic acid derivative CR2093 was intravenously administered to human gastric cancer-, rat pancreatic cancer R42J- or human large bowel cancer-bearing mice, proliferation of tumor was inhibited in the gastrin-stimulated group in which gastrin had been administered, but the proliferation of tumor was not inhibited in the gastrin-un-stimulated group (*Br. J. Cancer*, **65**, 879 - 883, 1992). In the same manner, also in the case of a benzodiazepine compound L-365260, inhibition of cancer cell proliferation was not found in the group of no gastrin stimulation (*Cancer*, **68**, 1255 - 1260, 1991). These facts mean that the selective gastrin/CCK-B receptor antagonists inhibited proliferation of cancer cells induced by gastrin only when gastrin stimulation was exogenously added, and no grounds are shown clinically that these selective gastrin/CCK-B receptor antagonists have antitumor effect in gastric cancer, pancreatic cancer, large bowel cancer and the like (that is, cases of no exogenous gastrin stimulation).

It has been reported that a non-selective gastrin/CCK receptor antagonist proglumide showed appropriate antitumor effect in large bowel cancer-bearing mice and reduced mortality rate of mice (ANN. Surg., 202, 303 - 9, 1985). However, since there are reports showing that CCK-A receptor is involved in gastrointestinal cancers (*Journal of Surgical Research,* **53** (3), 234 - 237 (1992); and *Cancer Letters,* **106** (2), 257 - 262 (1996)), it is not clear if the antitumor effect of proglumide mediates the CCK-A receptor or the gastrin/CCK-B receptor.

In addition, a selective gastrin/CCK-B receptor antagonist CI-988 as a non-benzodiazepine compound showed a growth inhibitory action without exogenous gastrin stimulation in human large bowel cancer-bearing mice at 25 mg/kg of oral administration, but the effect was not found at 50 mg/kg (Clinical and Experimental Pharmacology and Physiology, 23, 438 - 40, 1996).

As described in the above, role of the gastrin/CCK-B receptor in cancer has not been elucidated yet, and it was actually unclear if a selective gastrin/CCK-B receptor antagonist could become a clinically useful antitumor agent.

On the other hand, a 2,3,4,5-tetrahydro-1-(1H-imidazol-4-ylmethyl)-1H-1,4-benzodiazepine derivative has been reported as a benzodiazepine derivative having an antitumor action (J. Med. Chem., 43 (20), 3587, 2000), but its structure is completely different from that of the compound of this application.

## Disclosure of the Invention

The present inventors have conducted intensive studies on the physiological activity and action of the compound A and unexpectedly found as a result that the compound A ((3R)-N-(1-(1-tert-butylcarbonylmethyl)-2,3-dihydro-2-oxo-5-(2-pyridyl)-1H-1,4-benzodiazepin-3-yl)-N-(3-(methylamino)phenyl)urea) has good antitumor effects, particularly an excellent antitumor action upon pancreatic cancer and has reduced side effects even by a high dose administration, thereby accomplishing the invention.

Accordingly, the invention relates to an antitumor agent which contains the compound A or a salt thereof as the active ingredient. Preferably, it is an antitumor agent whose oral administration dose per day is from 0.1 to 1,000 mg/kg, more preferably 1 mg/kg or more, and an antitumor agent whose intravenous administration dose per day is from 0.01 to 100 mg/kg, more preferably 0.1 mg/kg. Also, the antitumor agent of the invention is desirable as a therapeutic agent of pancreatic cancer, large bowel cancer or gastric cancer, and it is particularly desirable a therapeutic agent of pancreatic cancer. In addition, the antitumor agent of the invention includes an antitumor agent to be used in a polypharmaceutical chemotherapy, namely an antitumor agent which is administered jointly with at least one other antitumor agent, simultaneously or at different times, with the same or different frequencies and by the same or different administration routes.

The invention also include use of the compound A or a salt thereof in producing an antitumor agent, particularly a medicament as an antitumor agent for use in polypharmaceutical chemotherapy, a method for treating a patient of cancer, which comprises administering an effective amount of the compound A or a salt thereof to the patient, and a method for treating a patient of cancer by polypharmaceutical chemotherapy, which comprises administering an effective amount of the compound A or a salt thereof and at least one other antitumor agent.

Best Mode for Carrying Out the Invention

The compound A or a salt thereof as the active ingredient of the invention can be synthesized by the aforementioned method described in *J. Med. Chem.*, 1997, **40** (3), 331 - 341, or WO 95/06040. In this connection, salts with inorganic acids (e.g., hydrochloride, sulfate, nitrate, acetate and the like) or organic acids can be exemplified as the salt of compound A.

The pharmaceutical composition of the invention can be prepared by a generally used method using pharmaceutical carriers, fillers and the like which are generally used in this field. The administration may be carried out by either oral administration using tablets, pills, capsules, granules, powders, solutions and the like, or parenteral administration using intravenous, intramuscular and the like injections.

As the solid composition for use in the oral administration according to the invention, tablets, powders, granules and the like are used. In such a solid composition, the active substances is mixed with at least one inert diluent such as lactose, mannitol, glucose, hydroxypropylcellulose, microcrystalline cellulose, starch, polyvinyl pyrrolidone, magnesium aluminate metasilicate or the like. In the usual way, the composition may contain other additives than the inert diluent, such as lubricant (e.g., magnesium stearate), a disintegrating agent, a stabilizing agent and a solubilization assisting agent. If necessary, tablets or pills may be coated with a sugar coat or a film of a gastric or enteric substance, such as sucrose, gelatin, hydroxypropylcellulose, hydroxypropylmethylcellulose phthalate or the like.

The liquid composition for oral administration includes pharmaceutically acceptable emulsions, solutions, suspensions, syrups, elixirs and the like and contains a generally used inert diluent such as purified water or ethyl alcohol. In addition to the inert diluent, this composition may also contain auxiliary agents such as a moistening agent, a suspending agent and the like, as well as sweeteners, flavors, aromatics and antiseptics.

The injections for parenteral administration includes aseptic aqueous or non-aqueous solutions, suspensions and emulsions. Examples of the diluent for use in the aqueous solutions and suspensions include distilled water for injection use and physiological saline. Examples of the diluent for use in the non-aqueous solutions and suspensions include propylene glycol, polyethylene glycol, plant oil (e.g., olive oil), alcohol (e.g., ethanol), polysorbate 80 (trade name) and the like. Such a composition may further contain auxiliary agents such as an antiseptic, a moistening agent, an emulsifying agent, a dispersing agent, a stabilizing agent and a solubilization assisting agent. These compositions are sterilized by filtration through a bacteria retaining filter, blending of a germicide or irradiation. Alternatively, they may be used by firstly making into sterile solid compositions and dissolving them in sterile water or a sterile solvent for injection prior to their use.

Clinical dose of the compound A in the medicament of the invention is optionally decided in response to each case by taking its administration method and symptoms, age, sex and the like of each patient into consideration. Generally, its daily dose is from 0.1 to 1,000 mg/kg, preferably from 0.5 to 500 mg/kg, more preferably from 1 to 100 mg/kg, in the case of oral administration, and the daily dose in the case of intravenous injection is approximately from 0.01 to 100 mg/kg, preferably from 0.1 to 10 mg/kg, and the daily dose is divided into 1 to several doses per day.

When the medicament of the invention is used in polypharmaceutical chemotherapy, it can be applied by adding it to a variety of the polypharmaceutical chemotherapy described in the aforementioned "Cancer Chemotherapy Handbook, Method for using antitumor agents and anti-side effects policy" or by substituting it with one or two antitumor agents described therein. Examples of the other antitumor agent which can be jointly used with this medicament preferably include antitumor agents which are used in the polypharmaceutical chemotherapy, such as antimetabolites (5-FU and derivatives thereof (tegafur, UFT, carmofur, doxifluridine, tegafur-gimestat and otastat potassium), GEM, methotrexate, 6-mercaptopurine, enocitabine, cytarabine and the like), carcinostatic antibiotics (MMC, ADM, epirubicin, aclarubicin, pirarubicin, THR, bleomycin, neocarzinostatin and the like), alkylating agents (nimustine, carboquone, ACNU, cisplatin, carboplatin and the like), carcinostatic plant alkaloids (vincristeine, vinblasteine, etoposide and the like), taxan (taxol and taxotere) and others (irinotecan, streptozocin and the like). More preferred are antimetabolites and taxan. Particularly preferred are 5-FU, GEM, taxol and taxotere known to have a pancreatic cancer therapeutic action.

Doses of the compound A and other antitumor agent to be jointly use are optionally decided for each drug in response to the drug to be used jointly, symptoms of each patient, administration method and the like. Dose of the compound A in the polypharmaceutical chemotherapy is as described in the foregoing. In addition, regarding the administration timing, administration frequency and administration route, most suitable method is selected for each drug to be used jointly. That is, the medicament of the invention is administered together with at least one (preferably one to four) of other antitumor agents, simultaneously or at different times, with the same or different frequencies and by the same or different administration routes. As the administration route of the compound A in polypharmaceutical chemotherapy, its one or two or more times of oral administration per day is desirable.

As will be shown later in Examples, it has been confirmed that when jointly used with other antitumor agents, the medicament of the invention can achieve excellent antitumor effects without increasing side effects. Accordingly, by introducing the medicament of the invention into polypharmaceutical chemotherapy, it is possible to reduce doses of other antitumor agents having strong side effects and it is expected that more superior antitumor effects can be obtained.

It has been reported that binding affinity of the compound A to the gastrin/CCK-B receptor is strong with a value ($IC_{50}$) of 0.1 nM, and it inhibits 50% of gastrin-stimulated gastric acid secretion in a dog at 0.01 mg/kg oral administration (*J. Med. Chem.*, 1997, **40** (3), 331-341). In contrast, the dose expressing the antitumor action of the invention is far higher dose, and it is unclear if the action mediates the gastrin/CCK-B receptor.

Antitumor actions and toxicity of the compound A are shown by the following Examples 1 to 5. Also, a preparation example of the antitumor agent of the invention is shown in Example 6.

Example 1 (Antitumor action in mice bearing various cancers)

Three to 10 x $10^6$ cells of human pancreatic cancer cell lines (BxPC-3 and PANC-1), human large bowel cancer cell lines (HT29 and COL0320DM) and a human gastric cancer cell line (MKN28) were injected into the dorsal side skin of female Balb/c nude mice. Starting on the next day of the subcutaneous injection of cancer cells, 200 mg/kg per day of the compound A was suspended in 0.5% methylcellulose and orally administered every day by dividing the daily dose into two doses per day. Also, 0.5% methylcellulose solution was administered in the same schedule to the control group. Using calipers, the tumor diameter was measured periodically until the next day of the final administration. The tumor volume and tumor growth inhibition ratio were calculated by the following calculation formulae.

$$\text{Tumor volume (mm}^3\text{) = 1/2 x breadth (mm)}^2 \text{ x length (mm)}$$

Tumor growth inhibition ratio (%) = [(tumor volume of control group - tumor volume of compound A administration group)/tumor volume of control group] x 100

The results are shown in the following table.

Table 1

| Cell line | Administration period | Tumor growth inhibition ratio |
|---|---|---|
| BxPC-3 | 23 days | 65% |
| PANC-1 | 49 days | 52% |
| HT29 | 27 days | 36% |
| COL0320DM | 21 days | 47% |
| MKN28 | 20 days | 31% |

It was confirmed that the medicament of the invention has good antitumor action upon human pancreatic cancer, human large bowel cancer and human gastric cancer.

Example 2 (Antitumor action and toxicity in BxPC-3 cancer-bearing mice)

In human pancreatic cancer cell BxPC-3 bearing mice, administration of the compound A was started when the tumor volume reached to about 120 $mm^3$ in a similar manner to that described in the test example 1. Its dose per day was 200 or 600 mg/kg, and the administration period was 21 days.
The tumor growth inhibition ratio was calculated by the following calculation formula.
Tumor growth inhibition ratio (%) = {[(tumor volume of control group on the administration-finished day - tumor volume of control group before commencement of the administration) - (tumor volume of compound A-treated group on the administration-finished day - tumor volume of compound A-treated group before commencement of the administration)]/(tumor volume of control group on the administration-finished day - tumor volume of control group before commencement of the administration)} x 100
The tumor growth inhibition ratio at the dose of 200 or 600 mg/kg was 48% or 79%, respectively, confirming dose-dependent antitumor action. In addition, changes in body weight in this test are shown in the following table. The compound A did not exert influence on the mouse body weight even when it was administered at a daily dose of 600 mg/kg continuously for 21 days, and side effects were not observed too. Accordingly, it was confirmed that the compound A can become a good antitumor agent with less side effects even at a high dosage.

Table 2

| Measured day | Body weight (g) | | |
|---|---|---|---|
| | Control group | Group of 200 mg/kg dose | Group of 600 mg/kg dose |
| The day before commencement of administration | 20.4 ± 0.7 | 22.1 ± 0.5 | 21.4 ± 0.6 |
| On the 21st day | 22.8 ± 0.7 | 23.8 ± 0.5 | 23.3 ± 0.7 |

Example 3 (Comparison with other selective gastrin/CCK-B receptor antagonist)

In human large bowel cancer cell COL0320DM bearing mice, the administration of compound A was started when the tumor volume reached to about 120 $mm^3$ in a similar manner to that described in the Example 2. Its dose per day was 60 or 200 mg/kg, and the administration period was 10 days. As a comparative compound, (3RS) -N-(1-(1-tert-butylcarbonylmethyl)-2,3-dihydro-2-oxo-5-(2-pyridyl)-lH-1,4-benzodiazepin-3-yl)-N'-(3-(dimethylamino) phenyl)urea whose data on inhibitory effect of pentagastrin-stimulus gastric acid secretion in a dog are disclosed as "compound of Example 38" in the aforementioned WO 93/16999 was administered in the same manner at a dose of 200 mg/kg.
The tumor growth inhibition ratio was calculated in a similar manner to that described in Example 2. The tumor growth inhibition ratio when 60 or 200 mg/kg of the compound A was administered was 20 or 27%, respectively. On the other hand, the comparative compound did not show the tumor growth inhibitory action.
The selective gastrin/CCK-B receptor antagonist disclosed in the prior art did not show the antitumor action under such a condition that the compound A of the invention showed the antitumor action.

Example 4 (Test on the comparison with Gemcitabine)

A total of 3 x $10^6$ cells of a human pancreatic cancer cell line BxPC-3 was injected into the dorsal side skin of female Balb/c nude mice (6 weeks of age). When the tumor volume reached to about 110 $mm^3$, 60, 200 or 600 mg/kg per day of the compound A was orally administered in a similar manner to that described in Example 2, every day by dividing the daily dose into two doses per day (n = 5). Also, 0.5% methylcellulose solution was administered in the same schedule to the control group. The administration period was 14 days.
As a comparative compound, a known pancreatic cancer therapeutic agent Gemcitabine (GEM) was used in the same test. GEM was intravenously administered at a dose of 60, 120 or 240 mg/kg per day, once in three days. Physiological saline was administered to the control group in the same schedule.
The tumor growth inhibition ratio was calculated by same calculation formula in Example 2.
The tumor growth inhibition ratio in the compound A-treated group is shown in Table 3, and that in the GEM-treated group in Table 4, together with various parameters which indicate the degree of side effects.

Table 3

| | Control | Compound A 60 mg/kg p.o. | Compound A 200 mg/kg p.o. | Compound A 600 mg/kg p.o. |
|---|---|---|---|---|
| Tumor growth inhibition ratio | - | 19.3 ± 23.6% | 25.1 ± 21.1% | 78.2 ± 28.3% |
| Body weight gain (Δ g) | +3.5 ± 0.3 | +3.5 ± 0.2 | +3.4 ± 0.2 | +3.6 ± 0.2 |
| Spleen weight (mg) | 97.9 ± 6.8 | 88.8 ± 9.2 | 88.6 ± 2.5 | 102.7 ± 6.5 |
| Kidney weight (mg) | 284.0 ± 14.2 | 293.8 ± 4.3 | 286.4 ± 7.1 | 296.2 ± 3.7 |
| Liver weight (g) | 1.34 ± 0.05 | 1.38 ± 0.03 | 1.32 ± 0.04 | 1.37 ± 0.02 |
| Erythrocyte count ($10^4$/μl) | 890.8 ± 36.4 | 902.2 ± 28.1 | 906.0 ± 14.3 | 956.2 ± 12.7 |

Table 4

| | Control | GEM 60 mg/kg i.v. | GEM 120 mg/kg i.v. | GEM 240 mg/kg i.v. |
|---|---|---|---|---|
| Tumor growth inhibition ratio | - | 22.2 ± 26.7% | 80.2 ± 20.1% | 3/5 dead cases |
| Body weight gain (Δ g) | +4.0 ± 0.4 | +2.7 ± 0.3 | +2.8 ± 0.7 | - |
| Spleen weight (mg) | 103.7 ± 7.7 | 111.7 ± 8.6 | 124.4 ± 12.1 | - |
| Kidney weight (mg) | 306.6 ± 8.4 | 284.8 ± 9.3 | 298.9 ± 14.3 | - |
| Liver weight (g) | 1.43 ± 0.05 | 1.21 ± 0.04* | 1.27 ± 0.06 | - |
| Erythrocyte count ($10^4$/μl) | 903.0 ± 27.5 | 795.8 ± 20.6 ** | 734.6 ± 3.9*** | - |

* P<0.05, ** P<0.01, *** P<0.001 vs. control group (Dunnett test)

The compound A showed a dose-dependent antitumor action in the BxPC-3-bearing model. In this case, it did not affect the body weight, the weight of organs (the spleen, kidney and liver) and the erythrocyte count. In addition, it did not affect other hematological parameters too (leukocyte count and platelet count).

On the other hand, since dead cases were observed in the GEM 240 mg/kg i.v. group, its MTD (maximum tolerated dose) was 120 mg/kg. GEM showed a dose-dependent antitumor action in the BxPC-3-bearing mice, and it also dose-dependently and significantly suppressed erythrocyte count. In addition, though slight, inhibition of body weight gain and reduction of liver weight were also found.

It was confirmed that the compound A of the invention can become a useful antitumor agent, because it has a good antitumor action similar to the known antitumor agent GEM, and its side effects are not particularly found.

Example 5 (Test on joint use with Gemcitabine)

In the same schedule as the case of Example 4, the compound A was orally administered every day at a dose of 200 or 600 mg/kg per day, and GEM was intravenously administered once in three days at a dose of 60 or 120 mg/kg (n = 5).

The results are shown in the following table.

Table 5

| | Compound A 200 mg/kg + GEM 60 mg/kg | Compound A 600 mg/kg + GEM 120 mg/kg |
|---|---|---|
| Tumor growth inhibition ratio | 93.4 ± 16.3% | 104.0 ± 15.2% a) |
| Body weight gain (Δ g) | +2.5 ± 0.3 | +1.6 ± 0.4 |

a): An inhibition ratio of 100% or more means regeression of tumor

Table 5 (continued)

| | Compound A 200 mg/kg + GEM 60 mg/kg | Compound A 600 mg/kg + GEM 120 mg/kg |
|---|---|---|
| Spleen weight (mg) | 124.7 ± 10.1 | 113.5 ± 5.4 |
| Kidney weight (mg) | 287.4 ± 13.3 | 273.5 ± 22.2 |
| Liver weight (g) | 1.31 ± 0.06 | 1.13 ± 0.05 |
| Erythrocyte count ($10^4$/μl) | 772.6 ± 24.4 | 706.2 ± 18.7 |

In the combined treatment group of 200 mg/kg of compound A and 60 mg/kg of GEM, its antitumor action (93.4%) was markedly excellent in comparison with the single 60 mg/kg GEM-treated group (22.2%) and the single 200 mg/kg compound A-treated group (25.1%). In addition, this action was more superior to the group in which 120 mg/kg of GEM, as its MTD, was administered (80.2%). Regarding side effects in this case, no changes were found in comparison with those of the single 60 mg/kg GEM-treated group, so that it was confirmed that their side effects were identical in spite of the markedly improved antitumor action by the combined use. Also, in the combined treatment group of 600 mg/kg of compound A and 120 mg/kg of GEM, the antitumor action was further improved and a growth inhibition ratio of 100% or more, namely regression of tumor, was observed. Regarding side effects, there was a tendency to reinforce the reduction of erythrocyte count, body weight gain and liver weight found in the single GEM-treated group, but serious side effects such as death were not found.

The above results show that by the combined use of the compound A with GEM, side effects were reduced and excellent antitumor effect was achieved, and the tumor regression which could not be obtained by the administration of GEM alone was also achieved.

Example 6 (Preparation of 100 mg tablets)

Using a fluidized granulation coating apparatus (manufactured by Okawara Mfg. Co., Ltd.), 200 g of the compound A, 146 g of lactose and 36 g of corn starch were uniformly mixed. A 90 g portion of 10% hydroxypropylcellulose solution was sprayed thereto to give granules. After drying, they were passed through a 20 mesh screen, mixed with 7.6 g of carboxymethylcellulose calcium and 1.4 g of magnesium stearate and then applied to a rotary tablet making machine (manufactured by Hata Seisakusho K.K.) to give tablets of 200 mg per tablet using a die-punch system of 8 mm x 9 R.

Industrial Applicability

Since it has been confirmed that the medicaments of the invention favorably inhibit the proliferation of cancer in various cancer-bearing mice and have a low toxicity, they are particularly useful as antitumor agents for pancreatic cancer, intestinal cancer, gastric cancer and the like. Particularly, since they have excellent antitumor action upon intractable pancreatic cancer, they are useful in treating pancreatic cancer. Also, since the medicaments of the invention have low toxicity, their high dose administration is also possible. In addition, since they can be administered orally, their administration route is also simple.

In case of using the medicaments of the invention in polypharmaceutical chemotherapy, administration dose of an antitumor agent having strong side effects can be lowered, so that realization of a polypharmaceutical chemotherapy achieving a favorable antitumor effect and relieved side effects is expected.

## Claims

1. An antitumor agent which comprises (3R)-N-(1-(1-tert-butylcarbonylmethyl)-2,3-dihydro-2-oxo-5-(2-pyridyl)-1H-1,4-benzodiazepin-3-yl)-N'-(3-(methylamino)phenyl)urea or a salt thereof as the active ingredient.

2. The agent according to claim 1, wherein it is an agent for treating pancreatic cancer, intestinal cancer or gastric cancer.

3. The agent according to claim 2, wherein it is an agent for treating pancreatic cancer.

4. The agent according to claims 1 to 3, wherein it is administered jointly with at least one other antitumor agent,

simultaneously or at different times, with the same or different frequencies and by the same or different administration routes.

5. Use of (3R)-N-(1-(1-tert-butylcarbonylmethyl)-2,3-dihydro-2-oxo-5-(2-pyridyl)-1H-1,4-benzodiazepin-3-yl)-N'-(3-(methylamino)phenyl)urea or a salt thereof for producing a medicament which is an antitumor agent.

6. The use according to claim 5, wherein the medicament is an antitumor agent which is administered jointly with at least one other antitumor agent, simultaneously or at different times, with the same or different frequencies and by the same or different administration routes.

7. A method for treating a cancer patient, which comprises administering an effective amount of (3R)-N-(1-(1-tert-butylcarbonylmethyl)-2,3-dihydro-2-oxo-5-(2-pyridyl)-1H-1,4-benzodiazepin-3-yl)-N'-(3-(methylamino)phenyl)urea or a salt thereof to the patient.

8. The treating method according to claim 7, wherein it further comprises administering at least one other antitumor agent to the patient, simultaneously or at different times, with the same or different frequencies and by the same or different administration routes.

## INTERNATIONAL SEARCH REPORT

International application No.
PCT/JP02/04482

**A. CLASSIFICATION OF SUBJECT MATTER**
Int.Cl[7] A61K31/5513, A61P35/00//C07D401/04, C07M7:00

According to International Patent Classification (IPC) or to both national classification and IPC

**B. FIELDS SEARCHED**

Minimum documentation searched (classification system followed by classification symbols)
Int.Cl[7] A61K31/5513, A61P35/00, C07D401/04, C07M7:00

Documentation searched other than minimum documentation to the extent that such documents are included in the fields searched

Electronic data base consulted during the international search (name of data base and, where practicable, search terms used)
CAPLUS, REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X | WO 95/6040 A1 (Yamanouchi Pharm. Co., Ltd.), 02 March, 1995 (02.03.95), & GB 2282595 A & CA 2169089 A & AU 9474661 A & ZA 9406474 A & EP 715624 A1 & CN 1129442 A & JP 9-504005 A & FI 9600836 A & NO 9600747 A & US 5728829 A | 1-6 |
| X | WO 93/16999 A1 (Yamanouchi Pharm. Co., Ltd.), 02 September, 1993 (02.09.93), & GB 2264492 A & AU 9336391 A & EP 628033 A1 & JP 7-505121 A & NO 9403133 A & FI 9403941 A & US 5688943 A | 1-6 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 July, 2002 (24.07.02) | 06 August, 2002 (06.08.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/04482

C (Continuation). DOCUMENTS CONSIDERED TO BE RELEVANT

| Category* | Citation of document, with indication, where appropriate, of the relevant passages | Relevant to claim No. |
|---|---|---|
| Y | WO 98/2419 A1 (Chugai Seiyaku Kabushiki Kaisha), 22 January, 1998 (22.01.98), & AU 9734594 A & JP 10-77230 A | 1-6 |
| Y | WO 98/404 A1 (Rotta Research Laboratorium S.P.A), 08 January, 1998 (08.01.98), & CA 2259345 A & AU 9733448 A & EP 918754 A1 & JP 2000-515497 A & KR 2000022416 A & US 6075033 A | 1-6 |
| Y | WO 96/28416 A1 (Daiichi Pharm. Co., Ltd.), 19 September, 1996 (19.09.96), & CA 2214569 A & AU 9648913 A & EP 985660 A1 & NO 9704233 A & US 5919824 A | 1-6 |
| Y | JP 8-40908 A (Yamanouchi Pharm. Co., Ltd.), 13 February, 1996 (13.02.96), (Family: none) | 1-6 |

Form PCT/ISA/210 (continuation of second sheet) (July 1998)

**INTERNATIONAL SEARCH REPORT**

International application No.
PCT/JP02/04482

**Box I Observations where certain claims were found unsearchable (Continuation of item 1 of first sheet)**

This international search report has not been established in respect of certain claims under Article 17(2)(a) for the following reasons:

1. [X] Claims Nos.: 7, 8
because they relate to subject matter not required to be searched by this Authority, namely:
The inventions as set forth in claims 7 and 8 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)